(19) Europäisches Patentamt
European Patent Office
Office européen des brevets

(11) Veröffentlichungsnummer: **0 413 918 A2**

(12) # EUROPÄISCHE PATENTANMELDUNG

(21) Anmeldenummer: **90111817.4**

(22) Anmeldetag: **22.06.90**

(51) Int. Cl.5: **C07C 69/734**, C07C 67/343

(30) Priorität: **23.08.89 DE 3927761**

(43) Veröffentlichungstag der Anmeldung: **27.02.91 Patentblatt 91/09**

(84) Benannte Vertragsstaaten: **AT CH DE ES FR GB IT LI NL**

(71) Anmelder: **HÜLS AKTIENGESELLSCHAFT**
**Patentabteilung / PB 15 - Postfach 13 20**
**D-4370 Marl 1(DE)**

(72) Erfinder: **von Itter, Franz-Albert, Dr.**
**Auf dem Hügel 109**
**D-5300 Bonn 1(DE)**
Erfinder: **Steffen, Klaus-Dieter, Dr.**
**Röckelstrasse 36**
**D-5202 Hennef 1(DE)**

(54) **Verfahren zur Herstellung von Alkoxyalkylidenmalonsäureestern.**

(57)

2.1 Die Reaktion von Malonsäureestern und Orthocarbonsäureestern zu Alkoxyalkylidenmalonsäureestern wird in Gegenwart von Carbonsäuren oder Anhydriden im allgemeinen durch Metallsalze, vorwiegend durch Schwermetallsalze, katalysiert.
2.2 Erfindungsgemäß wird nun die Reaktion durch leicht abtrennbare, unlösliche Aluminiumsilikate katalysiert.
2.3 Herstellung von Alkoxyalkylidenmalonsäureestern.

EP 0 413 918 A2

# VERFAHREN ZUR HERSTELLUNG VON ALKOXYALKYLIDENMALONSÄUREESTERN

Die Erfindung betrifft ein Verfahren zur Herstellung von Alkoxyalkylidenmalonsäuredialkylestern durch Umsetzung von Malonsäureestern mit Orthocarbonsäuretrialkylestern. Alkoxyalkylidenmalonsäureester stellen wichtige Zwischenstufen für die Synthese verschiedener substituierter Chinoline dar, die als Pharmazeutika gegen Malaria oder bakterielle Infekte eingesetzt werden.

Die Darstellung der Alkoxyalkylidenmalonsäureester aus Malonsäureestern und Orthocarbonsäureestern ist bekannt. Die Kondensation gelingt nach L. Claisen, Berichte 26 , 2729 (1893), in Essigsäureanhydrid mit $ZnCl_2$ als Katalysator.

Seitdem sind verschiedene Verbesserungen dieses Herstellungsweges vorgestellt worden mit dem Ziel, die Ausbeuten und die Produktivität der Umsetzung zu steigern.

Nach DE 24 26 964 wird die Umsetzung von Malonsäuredialkylestern mit einem Überschuß an Orthoameisensäuretrialkylestern in Gegenwart unterstöchiometrischer Mengen an Essigsäureanhydrid und Lewis-Säuren wie Zink-, Aluminium- oder Eisensalzen als Katalysator durchgeführt. Für die Darstellung von Ethoxymethylenmalonsäurediethylester wird eine Ausbeute von 94 % d.Th. bezogen auf Malonester genannt. Die Herstellung von Methoxymethylenmalonsäuredimethylester gelingt unter analogen Bedingungen mit einer Ausbeute von 70 % d.Th. und bei einem Druck von 3 bar mit einer Ausbeute von 86 % d.Th. bezogen auf den Malonsäureester. Diese Ausbeuten sind nicht zufriedenstellend. Infolge des unvollständigen Umsatzes werden außerdem Reinheiten von nur 94 % erhalten.

Wird, wie in US 2 824 121 beschrieben, die Darstellung von Ethoxymethylenmalonsäurediethylester durch Reaktion von Malonsäurediethylester mit Orthoameisensäuretriethylester in Abwesenheit von Lewis-Säuren mit Essigsäure als einzigem Katalysator durchgeführt, wird bei einem Umsatz von nur 60 % eine Ausbeute von 91,8 %, be zogen auf umgesetzten Malonsäurediethylester erreicht.

In EP 0 152 319 wird die Reaktion von Malonsäurediethylester mit Orthoameisensäuretriethylester durch Salze von Cd, Hg, Bi und Mg katalysiert. Die gaschromatographisch bestimmten Ausbeuten an Ethoxymethylenmalonsäurediethylester sind sehr hoch. Sie entsprechen aber nicht den praktisch erzielbaren Ausbeuten bei der Isolierung des Produkts.

Die Cd-, Hg- und Bi-Salze führen außerdem zu schwermetallhaltigen Produktrückständen. Die Entsorgung dieser Rückstände wird zunehmend schwierig und kostenintensiv. Auch aus ökologischen Gesichtspunkten erscheint eine Substitution der genannten Metallsalze erstrebenswert. Die Kontaminierung der Alkoxyalkylidenmalonester mit solchen toxischen Metallsalzen kann den Einsatz als pharmazeutische Vorprodukte einschränken oder gar unmöglich machen.

Die bekannten Katalysatoren liefern zudem bei der Umsetzung des weniger aktiven Orthoameisensäuretrimethylesters mit Malonsäuredimethylester den Methoxymethylenmalonsäuredimethylester in nicht zufriedenstellenden Ausbeuten.

Es bestand daher die Aufgabe, ein Verfahren zur Herstellung von Alkoxyalkylidenmalonsäureestern zu entwickeln, das ohne den Einsatz schwermetallhaltiger Katalysatoren bei einfacher Reaktionsführung zu guten Ausbeuten und Umsätzen führt. Insbesondere sollten auch Ausbeute und Umsatz bei der Herstellung von Methoxymethylenmalonsäuredimethylester und Ethoxymethylenmalonsäurediethylester verbessert werden.

Die Aufgabe wird überraschend dadurch gelöst, daß man bei der Herstellung von Alkoxyalkylidenmalonsäureestern der Formel

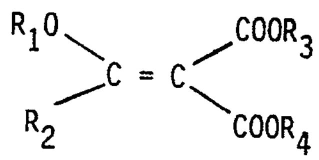

(1),

wobei

$R_1$ = Alkyl mit 1 bis 4 Kohlenstoffatomen,

$R_2$ = Wasserstoff, Alkyl mit 1 bis 4 Kohlenstoffatomen, Aryl, Aralkyl oder Alkoxy und

$R_3$, $R_4$ = Alkyl mit 1 bis 4 Kohlenstoffatomen ist,

aus Malonestern und Orthocarbonsäureestern in Gegenwart von Carbonsäuren oder deren Anhydriden Aluminiumsilikate als Katalysatoren einsetzt. Die Reste $R_1$, $R_3$ und $R_4$ können dabei gleich oder verschieden sein.

Die Erfindung betrifft vorzugsweise ein Verfahren zur Herstellung von Produkten der Formel (1), wobei $R_1$, $R_3$ und $R_4$ gleich sind und $R_2$ Wasserstoff oder Alkyl mit 1 bis 4 Kohlenstoffatomen ist. Ganz besonders betrifft die Erfindung die Herstellung von Methoxymethylenmalonsäuredimethylester und Ethoxymethylenmalonsäurediethylester.

Bei den als Katalysatoren eingesetzten, beanspruchten Aluminiumsilikaten handelt es sich bevorzugt um Ton-Minerale, sehr bevorzugt um Montmorillonit oder Montmorillonit enthaltende Schichtsilikate wie beispielsweise Bentonite, Smektite oder Attapulgit.

Zur Steigerung ihrer Aktivität können die genannten Katalysatoren mit Säuren oder sauren Salzen, bevorzugt mit Mineralsäuren wie beispielsweise Schwefelsäure oder Phosphorsäure behandelt werden. So können beispielsweise mit $H_2SO_4$ oder $H_3PO_4$ behandelte Bentonite verwendet werden. Eine Aktivierung der Katalysatoren kann auch während der Reaktion durch kontinuierliche oder portionsweise Zugabe der sauren Salze oder Säuren erfolgen. Aktivitätssteigerungen führen allgemein zu verbesserten Umsätzen.

Werden nur diese Säuren oder sauren Salze ohne die beanspruchten Katalysatoren eingesetzt, verlängert sich die erforderliche Reaktionszeiten beträchtlich bei gleichzeitig deutlich reduzierter Ausbeute.

Die Menge des eingesetzten Katalysators beträgt im allgemeinen 0,001 g bis 10 g pro Mol Malonat. Der Vorzugsbereich liegt bei 0,01 g bis 1 g pro Mol Malonat. Das den technischen Ton-Mineralien anhaftende Wasser kann durch übliche Trocknungsmethoden wie Erhitzen im Vakuum weitgehend entfernt werden. Da die bei der Reaktion eingesetzten Orthoester ebenfalls als Trockenmittel wirken, kann die technische Ware aber auch direkt verwendet werden.

Die Zugabe der beanspruchten Katalysatoren kann nach bekannten Methoden erfolgen.

Die beanspruchten Aluminiumsilikat-Katalysatoren können auch in Kombination mit üblicherweise verwendeten Metallsalz-Katalysatoren eingesetzt werden.

Die Reaktionstemperatur beträgt vorzugsweise 60 bis 200 °C. Ganz besonders wird ein Temperaturbereich von 100 bis 170 °C bevorzugt.

Bei der Reaktion wird vorzugsweise ein Druck von 1 bis 10 bar eingestellt. Ein Druck von 1 bis 3 bar wird besonders bevorzugt.

Die als weitere Kondensationsmittel eingesetzten Carbonsäuren und deren Anhydride sind vorzugsweise niedere aliphatische Carbonsäuren mit 1 bis 5 Kohlenstoffatomen bzw. die entsprechenden Anhydride. Die eingesetzte Menge liegt im allgemeinen bei 0,01 bis 1 Mol pro Mol Malonat.

Der Orthocarbonsäureester kann bei dem beanspruchten Verfahren stöchiometrisch oder im Überschuß verwendet werden. Die eingesetzte Menge beträgt vorzugsweise 1 bis 6 Mol pro Mol Malonsäureester. 1,2 bis 4 Mol Orthocarbonsäureester pro Mol Malonsäureester werden besonders bevorzugt.

Der Orthocarbonsäureester kann teilweise auch durch im selben Temperaturbereich siedende Lösemittel ersetzt werden. Derartige Lösemittel sind beispielsweise aromatische Kohlenwasserstoffe oder höher siedende Ether. Durch diese Lösemittel kann der Überschuß an Orthocarbonsäureestern etwas verringert werden.

Die Durchführung der Reaktion erfolgt am einfachsten, indem die Reaktanten und Katalysatoren in den obengenannten Verhältnissen zusammengegeben und bei den beschriebenen Temperaturen umgesetzt werden. Vorteilhaft werden Malonsäureester, Orthoester und der mineralische Katalysator mit einer Teilmenge Carbonsäure und/oder Anhydrid vorgelegt und die Restmengen Carbonsäure und/oder Anhydrid entsprechend dem Verlauf der Reaktion kontinuierlich oder portionsweise zugegeben. Es ist ebenfalls möglich, auch nur eine Teilmenge des Orthoesters mit den anderen Ausgangsverbindungen entsprechend den obengenannten Verfahren zur Reaktion zu bringen und den übrigen Teil kontinuierlich oder portionsweise während der Umsetzung nachzudosieren.

Der bei der Kondensation abgespaltene Alkohol wird vorteilhaft fortlaufend durch Destillation entfernt. Es kann sich empfehlen, nach Abreaktion des Malonsäureesters das Reaktionsgemisch noch solange bei der Reaktionstemperatur zu belassen, bis eine weitgehende Umwandlung der gebildeten Zwischenprodukte, wie der Bis-alkoxy-alkylmalonsäureester, in die Alkoxyalkylidenmalonsäureester erfolgt ist. Zur Vervollständigung der Reaktion können dem Reaktionsgemisch gegebenenfalls nach Abtrennung des mineralischen Katalysators weitere katalytische Mengen von 0,05 bis 0,5 Gew.-%, bezogen auf den eingesetzten Malonsäureester, an Carbonsäureanhydriden und/oder anorganischen oder organischen Säuren wie beispielsweise Schwefelsäure, Phosphorsäure, Citronensäure und p-Toluolsulfonsäure oder an sauren Salzen wie beispielsweise Kaliumhydrogensulfat zugesetzt werden. Das Gemisch wird dann 1 bis 5 Stunden bei 100 bis 180 °C getempert. Durch diese Maßnahme kann zum Beispiel die Produktreinheit von Methoxymethylenmalonsäuredimethylester auf über 98 % erhöht werden.

Das Verfahren kann bei Normaldruck und erhöhtem Druck durchgeführt werden. Da die besonders bevorzugten Reaktionstemperaturen im Bereich von 100 bis 170 °C liegen, die maximal erreichbare Reaktionstemperatur bei Normaldruck aber durch die Siedepunkte der Komponenten begrenzt ist, ermöglicht das Arbeiten unter Druck eine Erhöhung der Reaktionstemperatur auf die optimalen Werte und damit eine Steigerung der Reaktionsgeschwindigkeit. Dies führt zu deutlich verbes serten Raum/Zeit-Ausbeuten. Insbesondere bei Einsatz der relativ niedrig siedenden Orthoester, wie beispielsweise Orthoameisensäuretrimethylester oder Orthoessigsäuretrimethylester, lassen sich die Reaktions- und Nachreaktionszeiten verkürzen und in Folge verringerter Nebenproduktbildung die Ausbeute weiter steigern.

Nach Beendigung der Reaktion wird der überschüssige Orthoester bevorzugt durch Destillation bei vermindertem Druck abgetrennt. Dieser Orthoester kann weiteren Ansätzen wieder zugeführt werden.

Es ist vorteilhaft, vor der Destillation den Katalysator zu entfernen. Dabei weisen die erfindungsgemäßen Aluminiumsilikate Vorteile gegenüber den bisher verwendeten Metallsalzen auf, da sie unlöslich sind und ohne anderweitige Manipulationen problemlos durch Filtration oder Sedimentation abgetrennt werden können.

Abfiltrierte Aluminiumsilikat-Katalysatoren können außerdem nach Waschung mit dem entsprechenden Orthoester wieder eingesetzt werden. Dadurch vermindern sich die Stoff- und Entsorgungskosten.

Die erfindungsgemäßen Katalysatoren zeigen unter den Reaktionsbedingungen eine hohe Aktivität und Selektivität und erlauben einen praktisch quantitativen Umsatz der Malonsäureester. Es wird damit nunmehr möglich, auch Methoxymethylenmalonsäuredimetyhlester in Ausbeuten von über 92 % d.Th., bezogen auf eingesetzten Malonsäureester, zu erhalten. Bei der Synthese von Ethoxymethylenmalonsäurediethylester werden Ausbeuten von 95 % d.Th. an isoliertem Produkt erhalten. Es ist somit möglich, mit Hilfe von leicht abtrennbaren Aluminiumsilikaten die bisher verwendeten Schwermetallsalze zu substituieren.

## Beispiel 1

In einem Reaktionskolben, versehen mit Rührer, Thermometer, Tropftrichter, Kolonne und Rücklaufteiler, werden 264,2 g (2,0 m) Malonsäuredimethylester, 637 g (6 m) Orthoameisensäuretrimethylester und 20,4 g (0,2 m) Acetanhydrid mit 2 g mit $H_2SO_4$ aktivierter Bentonit (Montmorillonit-Katalysator KSF/O der Fa. Süd-Chemie, D-8000 München) vorgelegt und zum Sieden erhitzt. Während 6 Stunden werden kontinuierlich 40,8 g (0,4 m) Essigsäureanhydrid zudosiert. Der gebildete Leichtsieder wird bei einer Kopftemperatur von 62 bis 67 °C abdestilliert. Die Sumpftemperatur steigt dabei langsam von 109 bis auf 133 °C an. Nach 8 Stunden wird der überschüssige Orthoester bei vermindertem Druck abdestilliert. Das verbleibende Reaktionsgemisch wird mit 40,8 g (0,4 m) Acetanhydrid versetzt und 2 Stunden auf 140 °C erhitzt. Anschließend wird der Katalysator abfiltriert und das Rohprodukt im Vakuum destilliert. Bei 0,5 mbar und 110 °C Kopftemperatur werden 330,4 g Methoxymethylenmalonsäuredimethylester (98%ig) mit einem Schmelzpunkt von 44 bis 46 °C erhalten. Das entspricht einer Ausbeute von 92 % d.Th.

## Beispiel 2

In einem Glasautoklaven mit Druckdestillationsteil, Rührung, Temperaturmeßstellen, Sicherheitsventilen und Dosierpumpe werden 330,3 g (2,5 m) Malonsäuredimethylester, 1078 g (10,2 m) Orthoameisensäuretrimethylester, 15 g Acetanhydrid und 1 g Katalysator von Beispiel 1 eingefüllt. Der verschlossene Apparat wird auf 120 °C erhitzt, wobei sich ein Innendruck von 1,95 bar aufbaut. Dann werden im Verlauf von 5 Stunden 36 g Acetanhydrid in 3 g Portionen zudosiert. Die Innentemperatur steigt dabei langsam bis auf 155 °C an. Die gebildeten Leichtsieder destillierten bei 91 bis 92 °C Kopftemperatur ab.

Nach insgesamt 7,5 Stunden Reaktionszeit wird gekühlt, der Katalysator abfiltriert und der Orthoameisensäuretrimethylester-Überschuß bei leichtem Vakuum abdestilliert. Anschließend wird nach Zugabe von 3 Tropfen $H_3PO_4$ 2 Stunden auf 140 °C erhitzt und das Rohprodukt bei 0,5 mbar und 110 °C Kopftemperatur überdestilliert. Es resultieren 406 g Methoxymethylenmalonsäuredimethylester (98,6%ig), was einer Ausbeute von 91,9 % d.Th. entspricht.

## Vergleichsbeispiel A

Der Ansatz von Beispiel 2 wird wiederholt, wobei 0,25 g konz. $H_2SO_4$ als Katalysator eingesetzt werden und die Reaktionszeit auf 12 Stunden erhöht wird. Nach Destillation des Rohproduktes resultieren 360 g Methoxymethylenmalonsäuredimethylester mit einer Reinheit von 97 %, was einer Ausbeute von 80 % d.Th. entspricht.

## Beispiel 3

In der in Beispiel 1 beschriebenen Apparatur werden 160,2 g (1,0 m) Malonsäurediethylester, 474,2 g (3,2 m) Orthoameisensäuretriethylester, 4 g Acetanhydrid und 0,5 g Katalysator von Beispiel 1 vorgelegt und zum Sieden erhitzt. Im Verlauf von 6 Stunden wird ein Gemisch aus 10 g Acetanhydrid und 30 g Orthoameisensäuretriethylester kontinuierlich zugetropft. Der gebildete Leichtsieder wird bei 78 bis 79 °C Kopftemperatur abdestilliert. Die Reaktionstemperatur steigt dabei langsam bis auf 159 °C an. Das Gemisch wird eine weitere Stunde bei dieser Temperatur gehalten. Der nicht umgesetzte Orthoester wird anschließend bei vermindertem Druck abdestilliert, der Katalysator abfiltriert und das Rohprodukt durch Destillation bei 0,5 mbar gereinigt. Dabei werden 205,6 g Ethoxymethylenmalonsäurediethylester erhalten, was einer Ausbeute von 95,1 % d.Th. entspricht.

Beispiel 4

Der Ansatz von Beispiel 3 wird wiederholt, wobei als Katalysator 0,5 g mit $H_3PO_4$ aktivierter Bentonit (Montmorillonit-Katalysator KP 10 der Fa. Süd-Chemie, D-8000 München) eingesetzt werden. Nach Destillation werden 204,7 g Ethoxymethylenmalonsäurediethylester erhalten, was einer Ausbeute von 94,7 % d.Th. entspricht.

Beispiel 5

Der Ansatz von Beispiel 3 wird wiederholt, wobei als Katalysator 0,5 g Bentonit (Montmorillonit-Katalysator K 10 der Fa. Süd-Chemie, D-8000 München) eingesetzt werden. Nach Destillation werden Ethoxymethylenmalonsäurediethylester in einer Ausbeute von 76,8 % d.Th. und Diethoxymethylmalonsäurediethylester in einer Ausbeute von 18,2 % d.Th. erhalten.

Beispiel 6

160,2 g (1,0 m) Malonsäurediethylester, 474,2 g (3,2 m) Orthoameisensäuretriethylester und 0,5 g Katalysator von Beispiel 5 werden vorgelegt und zum Sieden erhitzt. Im Verlauf von 6 h wird eine Mischung aus 10 g Acetanhydrid, 1 g $H_3PO_4$ und 30 g Orthoameisensäuretriethylester kontinuierlich zugetropft. Die Umsetzung und Aufarbeitung erfolgt entsprechend Beispiel 3 und ergibt 200 g Ethoxymethylenmalonsäurediethylester, was einer Ausbeute von 92,6 % d.Th. entspricht.

Beispiel 7

In die in Beispiel 2 beschriebenen Druckapparatur werden 264 g (2 m) Malonsäuredimethylester, 961 g (8 m) Orthoessigsäuretrimethylester und 1 g Katalysator von Beispiel 1 eingefüllt. Es wird auf 130 °C aufgeheizt, wobei sich ein Innendruck von 1,5 bar einstellt. Dann werden 100 g Acetanhydrid in 12 Stunden eindosiert, die Leichtsieder bei 82 bis 87 °C Kopftemperatur abdestilliert und die Reaktionstemperatur allmählich auf 160 °C gesteigert. Nach beendeter Leichtsiederdestillation wird abgekühlt, der überschüssige Orthoessigsäuretrimethylester bei vermindertem Druck abdestilliert und das Rohprodukt im Hochvakuum von 0,3 mbar über eine kurze Kolonne bei 110 °C Kopftemperatur fraktioniert. Dabei werden 2-Carbmethoxy-3-methoxy- 2-butensäuremethylester in einer Ausbeute von 77,5 % d.Th. und 2-Carbmethoxy-3,3-dimethoxy-butansäuremethylester in einer Ausbeute von 11 % d.Th. erhalten.

Beispiel 8

In einer Glasapparatur mit Destillationsteil werden 160 g (1 m) Malonsäurediethylester, 649 g (4 m) Orthoessigsäuretriethylester und 0,5 g Katalysator von Beispiel 1 auf 130 °C aufgeheizt. Anschließend beginnt man, 102 g Acetanhydrid in 7 h zuzudosieren. Unter Destillation von Leichtsiedern bei 72 bis 73 °C Kopftemperatur wird die Sumpftemperatur bis auf 159 °C gesteigert. Nach Reaktionsende werden die restlichen Leichtsieder bei geringem Vakuum und der überschüssige Orthoessigsäuretriethylester bei 15 mbar abdestilliert. Das gewünschte Produkt wird danach bei 0,3 mbar und 100 bis 102 °C Kopftemperatur überdestilliert. 2-Carbethoxy-3-ethoxy-2-butensäureethylester wird dabei in einer Ausbeute von 73 % d.Th. erhalten.

Beispiel 9

In der in Beispiel 1 genannten Apparatur werden 188 g (1 m) Malonsäurediisopropylester, 571 g (3 m) Orthoameisensäuretriisopropylester und 0,05 g Katalysator von Beispiel 5 vorgelegt und auf 110 °C aufgeheizt. Dann werden innerhalb von 5 Stunden 102 g Acetanhydrid zudosiert. Die Sumpftemperatur wird auf 150 °C angehoben. Gleichzeitig wird unter Rückfluß eine Leichtsiederfraktion bei 60 °C Siedetemperatur abgenommen. Nach 10 Stunden läßt die Leichtsiederbildung nach. Nun wird zunächst bei 15 mbar und anschließend bei 0,3 mbar Vakuum fraktioniert. Es werden 7,6 % Malonsäurediisopropylester, 80,2 % Isopropoxymethylenmalonsäurediisopropylester und 6,5 Diisopropoxymethylmalonsäurediisopropylester erhalten. Die letztgenannte Verbindung wird durch Erhitzen mit 0,2 g $H_3PO_4$ in Isopropoxymethylenmalonsäurediisopropylester übergeführt.

**Ansprüche**

1. Verfahren zur Herstellung von Alkoxyalkylidenmalonsäuredialkylestern der Formel

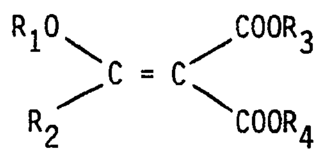

(1),

wobei
$R_1$ = Alkyl mit 1 bis 4 Kohlenstoffatomen,
$R_2$ = Wasserstoff, Alkyl mit 1 bis 4 Kohlenstoffatomen, Aryl, Aralkyl oder Alkoxy und
$R_3$, $R_4$ = Alkyl mit 1 bis 4 Kohlenstoffatomen ist,

durch Umsetzung von Malonsäuredialkylestern mit Orthocarbonsäuretrialkylestern in Gegenwart von Carbonsäuren und/oder deren Anhydriden,
dadurch gekennzeichnet,
daß als Katalysator Aluminiumsilikate eingesetzt werden.

2. Verfahren nach Anspruch 1,
dadurch gekennzeichnet,
daß $R_1$, $R_3$ und $R_4$ gleich sind und $R_2$ Wasserstoff oder Alkyl mit 1 bis 4 Kohlenstoffatomen ist.

3. Verfahren nach Anspruch 2,
dadurch gekennzeichnet,
daß $R_1$, $R_3$ und $R_4$ gleich sind und für Methyl oder Ethyl stehen und daß $R_2$ Wasserstoff ist.

4. Verfahren nach Anspruch 1,
dadurch gekennzeichnet,
daß als Aluminiumsilikate Ton-Minerale eingesetzt werden.

5. Verfahren nach Anspruch 4,
dadurch gekennzeichnet,
daß Montmorillonit oder Montmorillonit enthaltende Schichtsilikate eingesetzt werden.

6. Verfahren nach Anspruch 1,
dadurch gekennzeichnet,
daß mit Mineralsäuren und/oder sauren Salzen behandelte Aluminiumsilikate verwendet werden.

7. Verfahren nach Anspruch 6,
dadurch gekennzeichnet,
daß die zur Behandlung eingesetzten Mineralsäuren dem Reaktionsgemisch vor oder während der Reaktion kontinuierlich oder portionsweise zugefügt werden.

8. Verfahren nach Anspruch 1,
dadurch gekennzeichnet,
daß die Menge des eingesetzten Katalysators 0,001 g bis 10 g pro Mol Malonat beträgt.

9. Verfahren nach Anspruch 1,
dadurch gekennzeichnet,
daß die Temperatur der Reaktion bei 60 bis 200 °C gehalten wird.

10. Verfahren nach Anspruch 1,
dadurch gekennzeichnet,
daß die Umsetzung bei einem Druck von 1 bis 10 bar durchgeführt wird.

11. Verfahren nach Anspruch 1,
dadurch gekennzeichnet,
daß die Menge der eingesetzten Carbonsäuren und/oder deren Anhydride bei 0,01 bis 1 Mol pro Mol Malonat liegt.

12. Verfahren nach Anspruch 1,
dadurch gekennzeichnet,
daß die eingesetzte Menge an Orthocarbonsäuretrialkylester 1 bis 6 Mol pro Mol Malonat beträgt.

13. Verfahren nach Anspruch 1,
dadurch gekennzeichnet,
daß man nach der durch Aluminiumsilikate katalysierten Reaktion gegebenenfalls nach Abtrennung des Katalysators 0,05 bis 0,5 Gew.-%, bezogen auf den eingesetzten Malonsäureester, Carbonsäureanhydride und/oder anorganische oder organische Säuren oder saure Salze zusetzt und bei 100 bis 180 °C tempert.